# FASCICULE DE BREVET EUROPEEN

(11) **EP 0 904 040 B1**
(45) Date de publication et mention de la délivrance du brevet: **16.08.2000**
(21) Numéro de dépôt: 97926051.0
(22) Date de dépôt: 29.05.1997
(51) Int. Cl.: A61F 13/08

(54) **ORTHESE COMPRESSIVE DE TYPE BAS OU COLLANT DE CONTENTION**
KOMPRESIONSSTRUMPF ODER -STRUMPFHOSE
COMPRESSIVE SUPPORT ORTHOSIS STOCKING OR TIGHTS

(30) Priorité: 30.05.1996 FR 9606673
(43) Date de publication de la demande: 31.03.1999
(73) Titulaire: Innothera Topic International, 94110 Arcueil (FR)
(72) Inventeur: GARDON-MOLLARD, Christian, F-63400 Chamalières (FR)
(74) Mandataire: Dupuis-Latour, Dominique
(86) Numéro de dépôt international: FR9700934
(87) Numéro de publication internationale: WO9745081

(56) Documents cités:
- CH-A- 207 075
- DE-U- 8 814 047
- FR-A- 2 302 054
- FR-A- 2 433 935
- US-A- 3 983 870
- US-A- 4 048 818
- US-A- 4 351 068
- US-A- 4 513 740
- US-A- 5 497 513

## Description

L'invention a trait au domaine des orthèses compressives d'un ou des deux membres inférieurs, généralement connues sous la dénomination "bas de contention" ou "collants de contention".

Une orthèse de ce type est par example décrite dans le document US-A-4 513 740.

Bien que dans la suite on utilisera les termes "bas" ou "collant", l'invention n'est pas limitée à un article particulier, mais s'applique aussi bien à toutes les orthèses compressives, qu'il s'agisse de collants (couvrant les deux membres inférieurs et l'abdomen jusqu'à la ceinture, en une seule pièce), de mono-collants (collant muni d'une seule jambe, destiné à la contention d'un seul des membres inférieurs), de bas (couvrant la cuisse et le jarret) ou de chaussettes (couvrant le jarret seul).

Pour permettre une compression forte du ou des membres inférieurs, ces articles sont réalisés en un matériau élastique, typiquement une maille tricotée de texture très serrée, qui entraîne plusieurs séries de difficultés.

La première de ces difficultés, propre à tous les types de bas ou collants de contention, est celle de la difficulté de l'enfilage, en particulier au niveau du pied et de la cheville, avec un risque de mauvais placement, notamment à l'endroit du cou de pied et du talon qui sont des zones où l'enfilage est toujours assez délicat.

Une autre difficulté tient au fait que les zones de flexion (genou, cheville) sont le siège privilégié de plis et de strictions, inconvénient particulièrement sérieux pour les classes de contention les plus élevées (c'est-à-dire pour les mailles procurant les pressions les plus fortes). En effet, ces plis et strictions créent localement, au niveau des articulations, des pressions très fortes qui agissent comme des garrots aux points sensibles, allant ainsi à l'encontre du résultat recherché, qui est l'obtention d'un gradient de pression régulièrement dégressif de la cheville à la cuisse pour faciliter le retour veineux - situation qui n'est en fait correctement réalisée que pour une posture debout permanente immobile (sans flexion des articulations).

Si l'on prend en particulier le cas de la cheville, en position assise une flexion à cet endroit va entraîner une compression des vaisseaux, qui sont situés latéralement (à l'opposé du genou où ils passent sur la face postérieure de l'articulation, dans la région du creux poplité), avec, outre l'inconfort qui en résulte, risque de compression locale inopportune et très inconfortable, apparition d'oedème dans cette zone qui y est particulièrement sensible, etc.

L'invention a pour but de remédier à ces diverses difficultés en supprimant les strictions locales du matériel textile, en proposant un nouveau type d'orthèse compressive qui conserve néanmoins l'intégralité de l'effet thérapeutique recherché.

A cet effet, l'orthèse de l'invention, qui comprend une partie de jambe en maille compressive est caractérisée en ce qu'elle est essentiellement dépourvue de pied et de talon et en ce que l'extrémité inférieure de la partie de jambe comporte une bande élastique d'appui sur les malléoles, pourvue, sur sa face tournée vers la peau, d'un revêtement antidérapant.

Cet article présente, comme on l'expliquera plus en détail par la suite, des avantages de plusieurs ordres :
- médical : suppression des hyperpressions aux plis provoquées par les mouvements naturels de la cheville, tout en conservant l'effet thérapeutique recherché (pression dégressive depuis la cheville sur le membre inférieur) ;
- pratique : facilité d'enfilage du fait de l'absence de pied et de talon ;
- industriel : comme il n'est plus nécessaire de tricoter un gousset pour le talon et le pied, le processus de fabrication peut être grandement simplifié et accéléré, d'où une économie importante en termes de coût de fabrication.
- enfin, le confort est notablement augmenté du fait de l'absence de formation de plis à l'endroit de la cheville.

On va maintenant décrire un exemple de mise en oeuvre de l'invention, en référence aux dessins annexés.

La figure 1 représente, très schématiquement, un membre inférieur dans deux positions assises différentes.

La figure 2 est une vue d'ensemble de l'orthèse de l'invention.

La figure 3 est une vue agrandie, partiellement arrachée, de la partie inférieure de cette orthèse.

Les figures 4a à 4d illustrent diverses variantes de réalisation de la bande antidérapante.

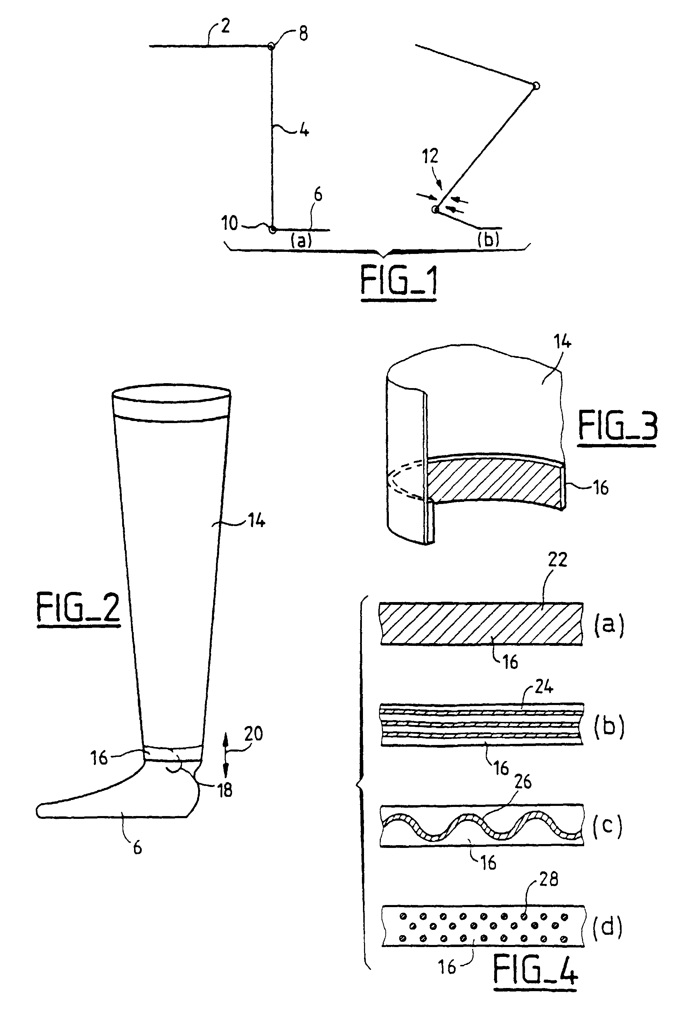

Sur la figure 1, on a schématisé en a un membre inférieur en position assise avec appui talonnier et, en b, en position assise sans appui talonnier. La référence 2 désigne la cuisse, la référence 4 la jambe (au sens strict, anatomique), la référence 6 le pied, la référence 8 l'articulation du genou et la référence 10 la cheville.

Dans la position de la figure 1a, la jambe et le pied sont sensiblement perpendiculaires, comme en position debout, ce qui correspond à la forme pour laquelle sont conçus les bas ou collants de contention.

En revanche, en position assise sans appui talonnier (figure 1b), il va se créer en 12, avec un bas ou un collant classiques, une zone de flexion, siège privilégié de plis et de strictions créant non seulement un inconfort (du fait des plis), mais également et surtout une compression excessive des vaisseaux qui, comme on l'a indiqué plus haut, sont, à la différence du genou, situés latéralement au niveau de la cheville avec risque d'oedème, de gêne et source d'inconfort.

La figure 2 illustre l'article de l'invention. Celui-ci comporte une partie de jambe 14 en maille compressive de type en elle-même classique, mais il est dépourvu de pied et de talon et il est arrêté en partie inférieure par une bande 16 pourvue d'un revêtement antidérapant venant prendre appui sur les malléoles 18 de la cheville, donc sur des saillies osseuses.

Il n'existe anatomiquement aucune compression vasculaire néfaste dans la zone d'appui de la bande 16, car les paquets vasculo-nerveux passent dans la gouttière rétro-malléolaire, supprimant ainsi tout risque de tension ou de striction dans la zone de l'articulation tibio-tarsienne.

La bande antidérapante 16 empêche le bas de la partie de jambe 14 de remonter (flèche 20), maintenant ainsi le bas ou le collant en place durant l'activité de la vie quotidienne. Le patient peut mettre une chaussette ordinaire par dessus le pied et la cheville, ou encore porter le bas ou le collant à la manière d'un caleçon.

L'effet thérapeutique recherché (pression dégressive depuis la cheville) est conservé en totalité, de sorte que les indications thérapeutiques du bas ou du collant de contention selon l'invention sont identiques à celles des bas ou collants classiques et ce, quelle que soit la classe de contention considérée.

Sur le plan de la réalisation, la partie de jambe 14 est réalisée en une maille identique à celle des bas classiques, par exemple les bas *Varisma* (marque déposée) d'Innothéra Topic. Les matériaux utilisés peuvent être un élasthanne guipé coton et polyamide, un élasthanne guipé polyamide sans coton, ou encore un mélange d'élasthanne et d'élasto-diène (latex de caoutchouc synthétique).

La maille est une maille classique, l'invention étant applicable à toutes les structures de mailles (tramée, jersey, côtes, micromesh pincée ou flottée, etc.), toutes connues en elle-mêmes du spécialiste des techniques de tricotage.

La bande antidérapante 16, que l'on a illustrée vue de l'intérieur sur la figure 3, est une bande légèrement élastique, c'est-à-dire que son élasticité dans le sens de la distension radiale est inférieure à celle de la partie de jambe, en effet, il ne s'agit pas de comprimer le membre à l'endroit des malléoles, mais simplement d'empêcher le bas de glisser vers le haut, ce qui est précisément obtenu sans serrage excessif par le matériau antidérapant.

Ce matériau est généralement un silicone, matière qui assure une bonne adhérence sur la peau, qui est anallergique et qui présente une bonne tenue dans le temps. D'autres matériaux présentant des propriétés semblables peuvent être employés, par exemple un latex, bien que ce matériau soit dans l'ensemble plus fragile que le silicone.

Le revêtement antidérapant peut être réalisé sous plusieurs formes, illustrées sur les figures 4a à 4d : Il peur s'agir d'un revêtement uniforme 22 de silicone, de bandes longitudinales 24, d'un tracé en zigzag 26 ou de gouttes 28. Ces types de bandes sont en elles-mêmes connus et sont par exemple disponibles auprès de Cheynet & Fils sous les références *Z327* (16 mm), *Z342* (16 mm) ou *0.5005* (15 mm).

Enfin, sur le plan industriel, on notera que la réalisation d'un bas ou d'un collant selon l'invention est fortement simplifiée dans la mesure où il n'y a plus à tricoter de gousset à l'endroit du talon et du pied.

Le bas se présente en effet sous la forme d'un simple tube légèrement conique, qui peut être tricoté très rapidement, donc avec une économie substantielle de temps et de moyens en termes de coût de fabrication.

Diverses variantes sont bien entendu envisageables sans sortir du cadre de l'invention pour mettre en oeuvre le principe d"'adhésivité distale" que l'on a exposé afin de permettre au collant de résister à la traction verticale de la maille sans pour autant serrer trop à la cheville.

Ainsi, la bande antidérapante 16 peut être une bande ajustable à la circonférence exacte de la cheville du patient, par exemple au moyen d'une bride ou d'un rabat maintenu à la position voulue par une bande agrippante.

De même, la bande antidérapante 16 n'est pas nécessairement une bande rapportée sur la partie de jambe 14, mais peut être constituée par l'extrémité de cette dernière, tricotée de façon à lui donner un caractère antidérapant sur sa face tournée vers la peau. Ceci peut par exemple être obtenu en incorporant à cet endroit, au tricotage, un fil tel qu'un élasthanne nu (non guipé) de fort titrage, qui va donner au collant un toucher rugueux et antidérapant côté peau à son extrémité distale.

## Revendications

1. Une orthèse compressive du type bas ou collant de contention, comprenant une partie de jambe (14) en maille compressive, caractérisée en ce qu'elle est essentiellement dépourvue de pied et de talon et en ce que l'extrémité inférieure de la partie de jambe comporte une bande élastique (16) d'appui sur les malléoles (18), pourvue, sur sa face tournée vers la peau, d'un revêtement antidérapant (22 ; 24 ; 26 ; 28).

## Patentansprüche

1. Komprimierende Orthese vom Typ Kompressionsstrumpf oder -strumpfhose, die einen Beinabschnitt (14) aus einem komprimierenden Maschenwerk aufweist, dadurch gekennzeichnet, daß sie im wesentlichen keinen Fuß und keine Ferse aufweist und daß das untere Ende des Beinabschnittes ein elastisches Stützband (16) über den Knöcheln (18) aufweist, das an seiner zur Haut hingewandten Seite mit einem Antirutsch-Überzug (22; 24; 26; 28) versehen ist.

## Claims

1. A compressive orthosis of the elastic stockings or tights type, comprising a leg portion (14) of compressive knit, characterized in that it is essentially lacking in any foot or heel portion and in that the bottom end of the leg portion includes an elastic cuff (16) for pressing against the malleoli (18) and provided on its face facing the skin with an antislip covering (22; 24; 26; 28).
